# EUROPEAN PATENT SPECIFICATION

(11) **EP 2 361 577 B1**
(45) Date of publication and mention of the grant of the patent: **23.10.2019**
(21) Application number: 11155976.1
(22) Date of filing: 25.02.2011
(51) Int. Cl.: A61B 18/08, A61B 17/29, A61B 18/14, A61B 17/00, A61B 34/00, A61B 17/064

(54) **Tension mechanism for articulation drive cables**
Spannmechanismus für Gelenkantriebskabel
Mécanisme de mise en tension pour câbles de commande d'articulation

(30) Priority: 26.02.2010 US 714086
(43) Date of publication of application: 31.08.2011
(73) Proprietor: Covidien LP, Mansfield, MA 02048 (US)
(72) Inventor: Cunningham, James S., Boulder, CO 80304 (US); Carlson, Richard, San Jose, CA 95112 (US); Jones, Eric, Livermore, CA 94550 (US)
(74) Representative: Soames, Candida Jane

(56) References cited:
- US-A- 4 688 555
- US-A1- 2001 041 893
- US-A1- 2006 020 287

## Description

### BACKGROUND

### 1. Technical Field

The present disclosure relates to an apparatus for surgically treating tissue. In particular, the disclosure relates to a mechanism for imparting a tensile force to cables extending through the apparatus.

### 2. Background of Related Art

Instruments such as electrosurgical forceps are commonly used in open and endoscopic surgical procedures to coagulate, cauterize and seal tissue. Such forceps typically include a pair of jaws that can be controlled by a surgeon to grasp targeted tissue, such as, e.g., a blood vessel. The jaws may be approximated to apply a mechanical clamping force to the tissue, and are associated with at least one electrode to permit the delivery of electrosurgical energy to the tissue. The combination of the mechanical clamping force and the electrosurgical energy has been demonstrated to join adjacent layers of tissue captured between the jaws. When the adjacent layers of tissue include the walls of a blood vessel, sealing the tissue may result in hemostasis, which may facilitate the transection of the sealed tissue. A detailed discussion of the use of an electrosurgical forceps may be found in U.S. Patent No 7,255,697 to Dycus et al.

Some endoscopic forceps are provided with a distal articulating portion to permit orientation of the jaws relative to a surgical site within the body of a patient. Mechanisms for articulating the distal end of an endoscopic instrument typically include a pair of drive cables or tensile members with distal ends anchored to the articulating portion on opposite sides of an instrument axis. The proximal ends of the drive cables are operatively coupled to an actuator that is responsive to an operator to draw one of the drive cables proximally while simultaneously permitting distal motion in the other drive cable. This motion in the drive cables induces pivotal motion of the distal end of the instrument. A surgical instrument comprising a housing having an elongated shaft according to the preamble of claim 1 is disclosed in US 2001/041893; a further example of such a surgical instrument is known from US 4,688,555.

The responsiveness of an articulating mechanism tends to be enhanced when the drive cables are configured to bear a tensile force. An adequate tensile force in the drive cables provides rigidity at the distal end of the instrument that permits a surgeon to perform procedures such as retraction and tissue tensioning. A drive cable under a tensile stress for a prolonged period is subject to creep deformation. Over extended periods of time, five years during storage of the instrument for example, a reduction of the tension in the drive cables may occur due to creep deformation. Accordingly, it may be beneficial to provide an apparatus to permit a variable force to be applied to drive cables over time to maintain the drive cables in a stressed condition.

### SUMMARY

The present disclosure describes a surgical instrument including a housing, an elongated shaft extending distally from the housing and an end effector extending distally from the elongated shaft. One or more tensile members extend at least partially through the elongated shaft. A distal end of one or more of the tensile members is operatively coupled to the end effector such that longitudinal motion in the tensile member induces movement of the end effector. A drive mechanism is operatively coupled to a proximal end of the tensile member to induce longitudinal motion in the tensile member. A tensioning mechanism is provides to impart a proximally directed force on the drive mechanism such that the proximally directed force is transmitted to the tensile member.

The tensioning mechanism may include a base hub coupled to the instrument in a fixed position relative to the tensile members. A spring may be coupled between the base hub and the drive mechanism to impart the proximally directed force on the drive mechanism.

The elongated shaft may include a proximal portion extending distally from the housing and a distal articulating portion extending distally from the proximal portion. The distal articulating portion may define a joint therein to permit the distal articulating portion to pivot with respect to the proximal portion of the elongated shaft. The tensile members may include a pair of articulation cables operatively coupled to the end effector such that relative longitudinal movement between the articulation cables induces articulation of the end effector. The drive mechanism may include first and second collars coupled to a respective articulation cable, and the spring may bear on the first collar.

The end effector may include a pair of jaw members, and the tensile member may be operable to move one or both of the jaw members between an open position substantially spaced from the other jaw member and a closed position wherein the jaw members are closer together. One or both of the jaw members may be coupled to a source of electrical energy.

According to another aspect of the disclosure, a surgical instrument includes a housing and an elongated shaft extending distally from the housing. The elongated shaft includes a proximal portion defining a longitudinal axis and a distal articulating portion that is pivotable with respect to the proximal portion. An articulation drive mechanism is provided to pivot the distal articulating portion of the elongated shaft. The articulation drive mechanism includes on or more tensile members extending at least partially through the elongated shaft. The tensile members are configured to induce the distal articulating portion of the elongated shaft to pivot. A tensioning mechanism is configured to impart a variable force to the tensile members to maintain the tensile members in tensile state. The variable force is dependent upon a variable length of one or more of the tensile members.

The tensioning mechanism may include a spring operatively coupled to the tensile members to transmit a force to the tensile members. The spring may be configured to change in length in response to a change in length of the tensile members. The spring may be a compression spring coupled between a stationary base hub and a movable component of the articulation drive mechanism to impart a variable force to the movable component. The movable component may be a first collar coupled to the at least one tensile member, and the first collar may be longitudinally movable to induce the distal articulating portion of the elongated shaft to pivot. The articulation drive mechanism may include a second collar longitudinally movable in response to pivotal motion of the distal articulating portion of the elongated shaft.

According to another aspect of the disclosure, a surgical instrument includes a housing, an elongated shaft extending distally from the housing and an end effector extending distally from the elongated shaft. One or more tensile members extend at least partially through the elongated shaft and are movable from the housing to induce movement of the end effector. A spring is operatively coupled to the tensile member to impart a tensile force thereto.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.

Reference(s) to "embodiment(s)" throughout the description which are not under the scope of the appended claims merely represent possible exemplary executions and are therefore not part of the present invention.
FIG. 1 is a perspective view of a surgical instrument in accordance with an embodiment the present disclosure;
FIG. 2 is an enlarged, perspective view of the area of detail identified in FIG. 1 depicting a distal articulating section of the instrument;
FIG. 3 is another perspective view of the distal articulating section of the instrument;
FIG. 4 is an exploded, perspective view of an articulation drive mechanism of the instrument;
FIG. 5A is a top view of the distal articulating section of the instrument in a neutral position;
FIGS. 5B is a top view of the distal articulating section of the instrument in an articulated position;
FIG. 6 is a schematic, top view of a tensioning mechanism of the instrument including a spring bearing on an articulation drive mechanism; and
FIG. 7 is a schematic top, view of an alternate embodiment of a tensioning mechanism including a spring bearing on a collar of an articulation drive mechanism.

### DETAILED DESCRIPTION

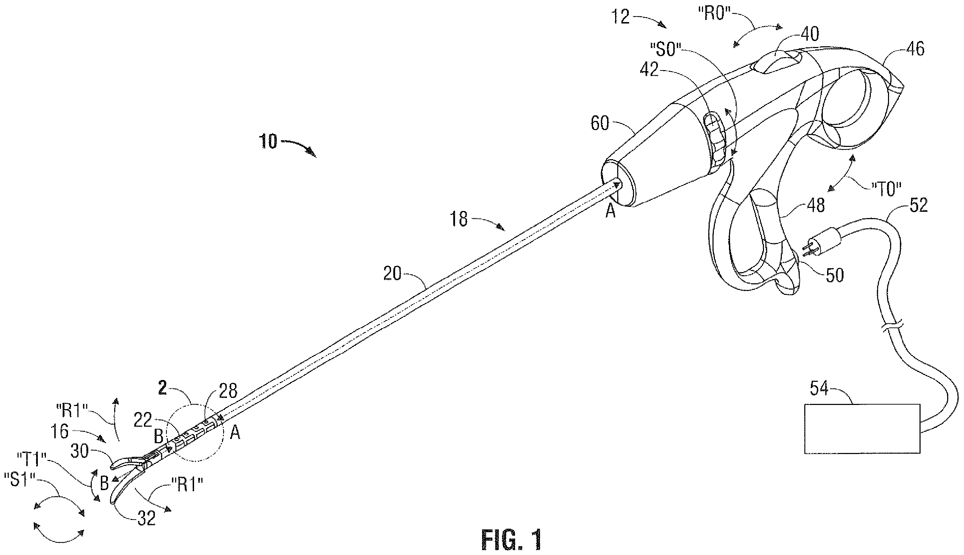

Referring initially to FIG. 1, an embodiment of an electrosurgical instrument is depicted generally as 10. The instrument 10 includes a housing 12 remotely supporting an end effector 16 through an elongated shaft 18. Although this configuration is typically associated with instruments for use in endoscopic surgical procedures, various aspects of the present disclosure may be practiced in connection with traditional open procedures as well.

Elongated shaft 18 includes a proximal portion 20 extending from the housing 12 and an articulating distal portion 22 supporting the end effector 16. The proximal portion 20 defines a longitudinal axis A-A, and is sufficiently long to position the end effector 16 through a cannula (not shown). The articulating distal portion 22 defines at least one joint 28 between the proximal portion 20 of the elongated shaft 18 and the end effector 16 permitting the end effector 16 to articulate or pivot relative to the longitudinal axis A-A. The end effector 16 defines an end effector axis B-B, which may be aligned with the longitudinal axis A-A to facilitate insertion of the end effector 16 through the cannula, and thereafter moved to orient the end effector 16 relative to a surgical site within the body of a patient.

The end effector 16 includes a pair of opposing jaw members 30 and 32. The jaw members 30, 32 are operable from the housing 12 to move between an open configuration to receive tissue, and a closed configuration to clamp the tissue and impart an appropriate clamping force thereto. When the end effector 16 is in the open configuration, a distal portion of each of the jaw members 30, 32 is spaced from the distal portion of the other of the jaw members 30, 32. When the end effector 16 is in the closed configuration, the distal portions of the jaw members 30, 32 are closer together. The end effector 16 is configured for bilateral movement wherein both jaw members 30 and 32 move relative to the end effector axis B-B as the end effector 16 is moved between the open and closed configurations. However, unilateral motion is also contemplated wherein one of the jaw members 30, 32, e.g., jaw member 32 remains stationary relative to the end effector axis B-B and the other of the jaw members 30, 32, e.g., jaw member 30, is moveable relative to the end effector axis B-B.

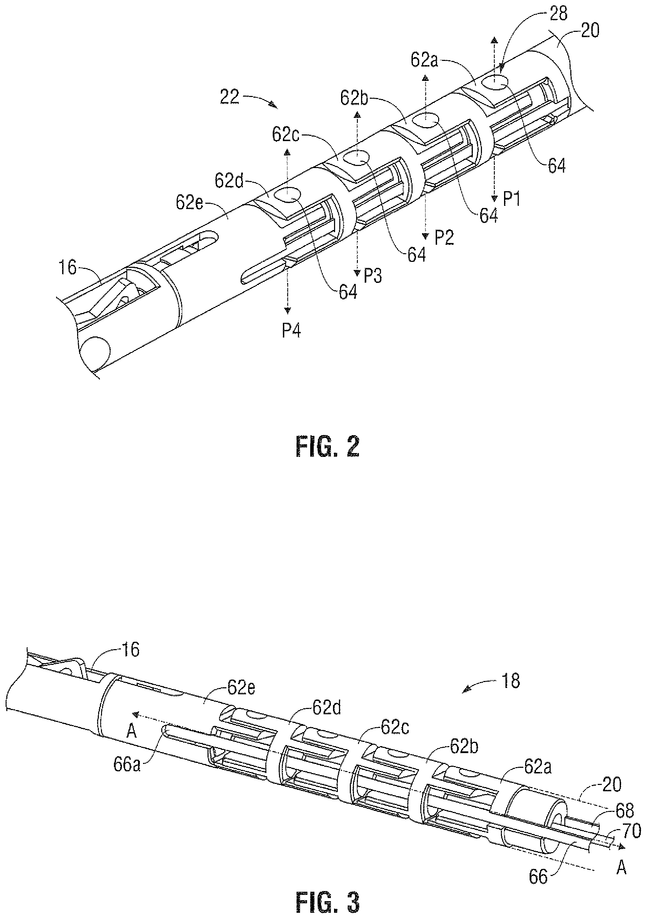

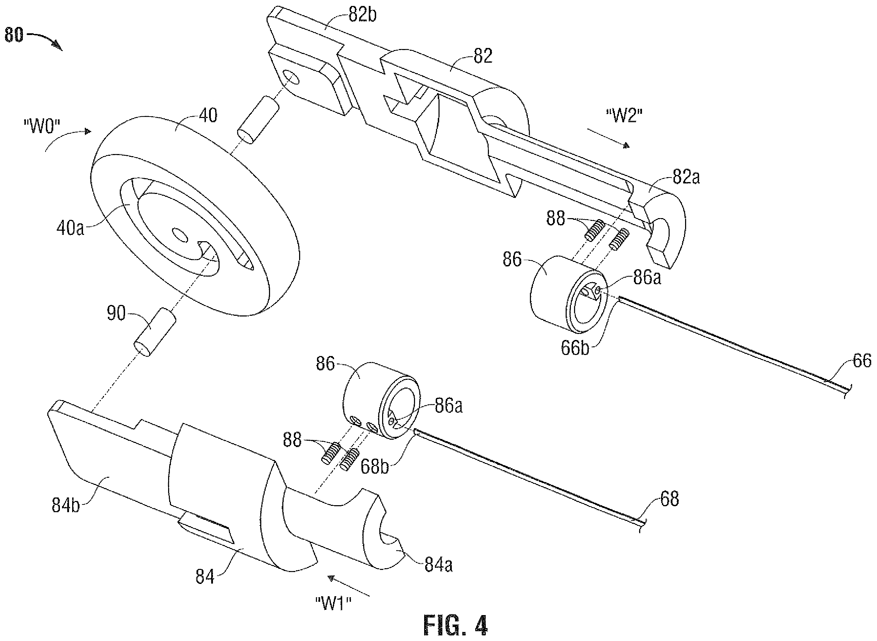

The housing 12 supports various actuators that are responsive to manipulation by an operator to induce these and other movements of the end effector 16. These actuators include an articulation wheel 40, which is operable to articulate the distal portion 22 of the elongated shaft 18 with respect to the longitudinal axis A-A. As described in greater detail below, the articulation wheel 40 is operatively coupled to the articulating distal portion 22 of the elongated shaft 18 by a pair of tensile members such as drive cables 66, 68 (see Figures 3 and 4) such that rotation of the articulation wheel 40 in the direction of arrows "R0" induces pivotal motion of the end effector 16 in the direction of arrows "R1" about the joints 28. The responsiveness of the end effector 16 to pivot upon rotation of the articulation wheel 40 is affected in part by a tensile force carried in the drive cables 66, 68 as described in greater detail below.

Other actuators supported by the housing 12 include a roll knob 42 and a movable handle 46. The roll knob 42 is operable to rotate the end effector 16 about the end effector axis B-B. Rotation of the roll knob 42 in the direction of arrow "S0" induces rotational motion of the end effector 16 in the direction of arrows "S1." The articulation wheel 40 and roll knob 42 cooperate to permit the end effector 16 to be appropriately positioned and oriented to effectively engage tissue. Once the end effector 16 is positioned and oriented, the surgeon may approximate the movable handle 46 relative to a stationary handle 48 to move the jaw members 30, 32 to the closed configuration. Separation of the movable handle 46 from the stationary handle 48 moves the jaw members 30, 32 to the open configuration. Thus, motion of the movable handle 46 in the direction of arrows "T0" induces motion in the end effector 16 in the direction of arrows "Tl."

The stationary handle 48 is provided with a power port 50 for receiving an electrosurgical cable 52. The cable 52 is in electrical communication with a source of electrosurgical energy such as electrosurgical generator 54. The electrosurgical generator 54 serves to produce electrosurgical energy and also to control and monitor the delivery of the electrosurgical energy to the instrument 10. Various types of electrosurgical generators 54, such as those generators provided by Covidien - Energy-based Devices, of Boulder, Colorado, may be suitable for this purpose. Electrosurgical generator 54 may include a foot pedal (not shown), or other actuator to initiate and terminate the delivery of electrosurgical energy to the instrument 10. The power port 50 on the stationary handle 48 is in electrical communication with at least one of the jaw members 30, 32 such that the electrosurgical energy supplied by the generator 54 may be delivered to tissue clamped in the end effector 16.

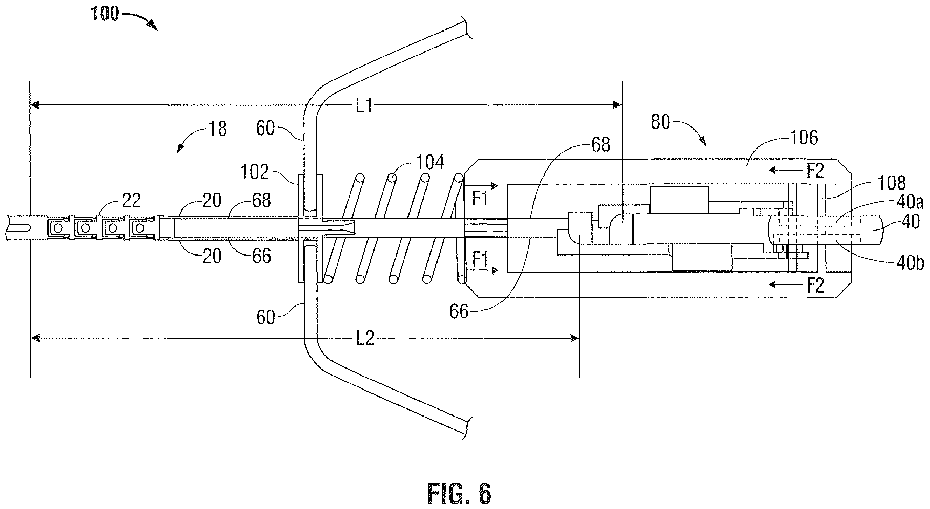

Instrument 10 is provided with a tensioning mechanism 100 for imparting a tensile force to the articulation drive cables 66, 68. The tensioning mechanism 100 is fixedly coupled to a housing member 60 of the stationary handle 48. The housing member 60 provides a stationary reference for the movable components of the tensioning mechanism 100 as described below with reference to FIG. 6.

Referring now to FIG. 2, the articulating distal portion 22 of the elongated shaft 18 includes a plurality of discrete links 62a, 62b, 62c, 62d and 62e. A proximal-most link 62a is fixedly coupled to the proximal portion 20 of the elongated shaft 18, and a distal-most link 62e supports the end effector 16. A plurality of intermediate links 62b, 62c, and 62d extend between the proximal-most link 62a and the distal-most link 62e. Each of the links 62a, 62b, 62c, 62d and 62e is pivotally coupled to at least one neighboring link 62a, 62b, 62c, 62d 62e by a pivot pin 64. The pivot pins 64 define four pivot axes P1, P2, P3 and P4 about which the neighboring links 62a, 62b, 62c, 62d and 62e may pivot to define the joints 28. In the embodiment depicted in FIG. 2, each of the pivot pins 64 are arranged in a substantially parallel manner such that the distal end 22 of the elongated shaft 18 is permitted to pivot in a single plane to orient the end effector 16. In other embodiments, pivot axes (not shown) may be oriented orthogonally or obliquely with respect to one another to permit the distal end to pivot in multiple planes. In still other embodiments, the at least one joint 28 may be defined with a flexible or bendable portion (not shown) of the elongated shaft 18.

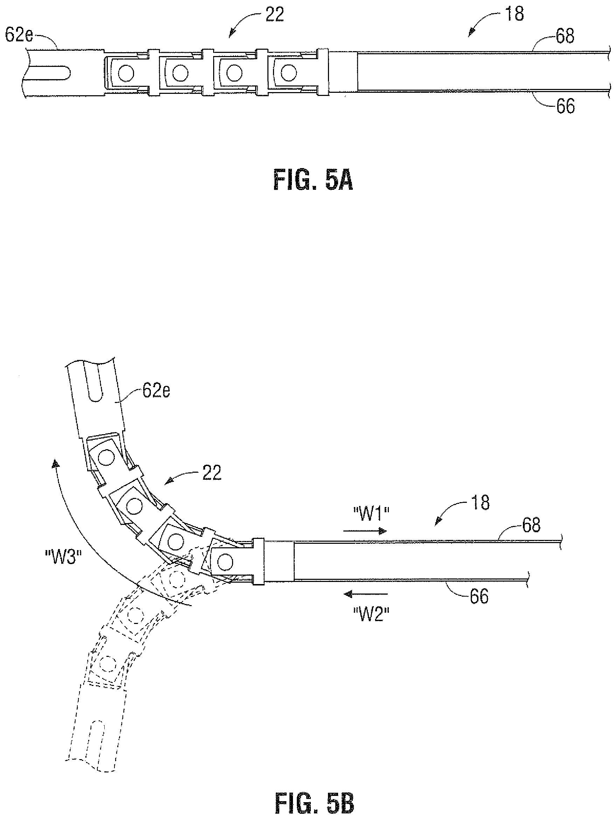

In order pivot the links 62a, 62b, 62c, 62d, 62e about the respective axes P1, P2, P3, P4, a pair of longitudinally extending and reciprocating drive cables 66 and 68 are provided as depicted in FIG. 3. A distal end 66a of the drive cable 66 is affixed to the distal-most link 62e on an opposite lateral side of the distal-most link 62e with respect to a distal end 68a (FIG. 6A) of drive cable 68. The drive cables 66, 68 extend from the distal-most link 62e proximally through the links 62d, 62c, 62b, 62a and through the proximal portion 20 of the elongated shaft 18 into the housing 12 (FIG. 1). In the housing 12, the articulation drive cables 66 and 68 are operatively associated with articulation wheel 40 as described below with reference to FIG. 5. Distal advancement of one of the drive cables 66 or 68 and simultaneous proximal retraction of the other of drive cables 66 or 68 function to cause links 62a, 62b, 62c, 62d and 62e to pivot relative to each other thereby causing a bend in articulating distal portion 22.

An additional tensile member such as drive cable 70 may extend through the elongated shaft 18. A distal end of the drive cable 70 may be operatively coupled to the end effector 16 to move the jaw members 30, 32 (FIG. 1) between the open and closed configurations. Longitudinal motion of the drive cable 70 may be translated into pivotal motion of the jaw members 30, 32 as described, for example, in U.S. Patent No 7,083,618 to Couture et al. A proximal end of the drive cable 70 may be operatively coupled to movable handle 46 (FIG. 1) such that longitudinal motion of the drive cable 70 may be induced by manipulation of the movable handle 46.

Referring now to FIG. 4, articulation mechanism 80 is depicted independent of the remaining instrument components. The articulation mechanism 80 includes a pair of shuttles 82, and 84 to advance and retract the drive cables 66, 68. Shuttles 82 and 84 are provided with distal hooks 82a and 84a, which engage and alternatively retract a pair of collars 86. Each of the collars 86 includes a bore 86a for receipt of a proximal end 66b, 68b of the drive cables 66, 68. Set screws 88 secure the drive cables 66, 68 within bores 86a.

Shuttles 82 and 84 have respective proximal ends 82b and 84b, which are configured to engage articulation wheel 40 with pins 90 extending therefrom. The pin 90 that extends from the proximal end 84b of shuttle 84 engages a spiral groove 40a inscribed into a lateral side of the articulation wheel 40. On an opposite lateral side of the articulation wheel 40, a second spiral groove 40b (FIG. 6) is inscribed in an opposite orientation and is engaged by the pin 90 extending from the proximal end 82b of the shuttle 82. The spiral grooves, e.g., groove 40a, permit rotational movement of the articulation wheel 40 to be translated into longitudinal and reciprocal motion of shuttles 82 and 84. Rotation of the articulation wheel 40 in the direction of arrow "W0" induces the shuttle 84 and the drive cable 68 to move in the direction of arrow "W1." Longitudinal motion of the drive cable 68 in the direction of arrow "W1" induces the distal portion 22 of the elongated shaft 18 to move from a straight configuration (FIG. 6A) to an articulated configuration in the direction of arrow "W3" (FIG. 6B). Rotation of the articulation wheel 40 in a direction opposite the direction of arrow "W0" induces an opposite motion such that the distal portion 22 of the elongated shaft 18 is articulated in an opposite direction as depicted in phantom in FIG. 6B.

It should be noted that, since the drive cables 66 and 68 are secured to the distal-most link 62e as described above, as one of the drive cables 66 or 68 is pulled proximally by respective hook 82a or 84a, the other of drive cables 66 or 68 is automatically drawn distally. Thus, there is no need for the shuttles 82, 84 to provide a structure for pushing or driving either of the collars 86 distally.

Referring now to FIG. 6, a tensioning mechanism 100 is provided to impart a tensile force on the articulation drive cables 66, 68. The tensioning mechanism 100 includes a base hub 102 that is fixedly coupled to the housing member 60 and provides a stationary reference or ground for the tensioning mechanism 100. The drive cables 66, 68 move freely through the base hub 102 as the articulation wheel 40 is manipulated to articulate the distal portion 22 of the elongated shaft 18. A compression spring 104 is mounted within the housing member 60 such that the spring 104 bears on the base hub 102 at distal end thereof, and bears on a carrier 106 at a proximal end thereof. The compression spring 104 provides a proximally directed force to the carrier 106 in the direction of arrow "F1." The carrier 106 is mounted within the housing member 60 such that minor adjustments to the longitudinal position of the carrier 106 may be achieved. An axle 108 couples the articulation drive mechanism 80 to the carrier 106. The tensile force imparted to the drive cables 66, 68 acts upon the axle 108 in the direction of arrows "F2." When the force imparted by the drive cables 66, 68 is balanced by the force of the spring 104, the carrier 106 remains stationary, and the articulation drive mechanism 80 may be operated as described above with reference to Figures 4, 5A and 5B.

Over time, the drive cables 66, 68 may experience fatigue or slight deformations associated with bodies subject to prolonged stress. For example, prolonged exposure to the tensile stress imparted to the drive cables 66, 68 may result in an increase in a respective length L1, L2 of each of the drive cables 66, 68. When L1 and L2 increase, the carrier 106 will move proximally under the influence of the spring 104. This movement compensates for the change in the respective length of the drive cables 66, 68, and thus, the drive cables 66, 68 remain in tension. In this way, the tensioning mechanism 100 imparts a tensile force to the drive cables 66, 68 and maintains operability and responsiveness of the articulation mechanism 80.

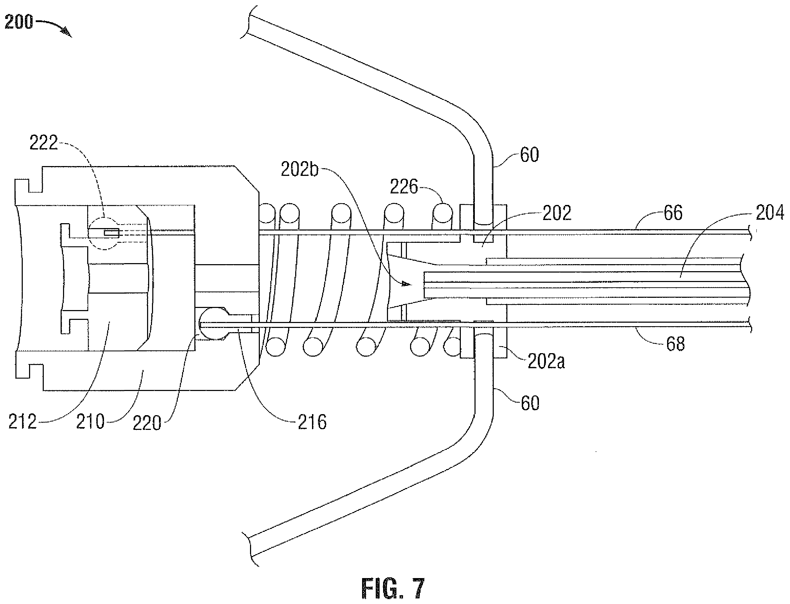

Referring now to FIG. 7, an alternate embodiment of a tensioning mechanism 200 includes a stationary base hub 202. The base hub 202 includes flanges 202a that are fixedly coupled to housing member 60, and a central opening 202b that permits the passage of various reciprocating or rotating actuation members 204 therethrough. The actuation members 204 may be operable to induce movements of the end effector 16 such as opening and closing the jaw members 30, 32 (FIG. 1). Openings in the flanges 202a are provided to permit free passage of the articulation drive cables 66, 68 therethrough.

The articulation drive cables 66, 68 are each coupled to a respective collar 210, 212. A proximal end of drive cable 68 is fixedly coupled to an anchor 216. The anchor 216 is disposed within a tapered slot 220 of outer collar 210 and maintained therein by a tensile force imparted to the drive cable 68. A proximal end of drive cable 66 is similarly coupled to anchor 222 and held within inner collar 212 by a tensile force imparted to the drive cable 66. The inner collar 212 is nested within outer collar 210, and is longitudinally movable therein. An actuator (not shown) may be provided to induce opposed longitudinal motion between the two collars 210, 212 to induce induce articulation in the end effector as described above in FIG. 5B.

A spring 226 bears on the stationary base hub 202 and exerts a proximally directed force on the outer collar 210. This force is transmitted to the drive cables to maintain a constant tension on the drive cables 66, 68 despite creep elongation of the cables 66, 68, or any tolerance stack-up that may occur. The deflection, spring constant, or other feature of spring 226 may be selected to provide an appropriate tension to the drive cables 66, 68. The number of springs may also be adjusted.

Although the foregoing disclosure has been described in some detail by way of illustration and example, for purposes of clarity or understanding, it will be obvious that certain changes and modifications may be practiced within the scope of the appended claims.

## Claims

1. A surgical instrument (10), comprising:
a housing (12) having an elongated shaft (18) extending distally therefrom, the elongated shaft supporting an end effector (16) for treating tissue;
at least one tensile member (66, 68) extending at least partially through the elongated shaft, a distal end of the at least one tensile member operatively coupled to the end effector such that longitudinal motion in the at least one tensile member induces movement of the end effector;
a drive mechanism (80) operatively coupled to a proximal end of the at least one tensile member, the drive mechanism including an actuator (40) operable to induce longitudinal motion in the at least one tensile member; and
a tensioning mechanism configured to constantly impart a proximally directed force on the drive mechanism such that the proximally directed force is transmitted to the at least one tensile member, **characterized in that**
the elongated shaft includes a proximal portion (20) extending distally from the housing and a distal articulating portion (22) extending distally from the proximal portion, the distal articulating portion defining at least one joint therein to permit the distal articulating portion to pivot with respect to the proximal portion of the elongated shaft,
the at least one tensile member includes a pair of articulation cables (66, 68) operatively coupled to the end effector such that relative longitudinal movement between the articulation cables induces articulation of the end effector,
the end effector includes a pair of jaw members (30, 32),
the surgical instrument further includes a drive cable (70) extending at least partially through the elongated shaft and operable to move at least one of the pair of jaw members between an open position wherein the pair of jaw members are substantially spaced from each other and a closed position wherein the pair of jaw members are closer together, and
the proximally directed force of the tensioning mechanism is transmitted to the pair of articulation cables to maintain a constant tension on the pair of articulation cables despite any creep elongation of the pair of articulation cables.

2. The surgical instrument according to claim 1, wherein the tensioning mechanism includes a base hub (102) coupled to the instrument in a fixed position and a spring (104) coupled between the base hub and the drive mechanism to impart the proximally directed force on the drive mechanism.

3. The surgical instrument according to claim 1 or 2, wherein the drive mechanism includes first and second collars (210, 212) coupled to a respective articulation cable, and wherein the spring bears on the first collar.

4. The surgical instrument according to claim 1, 2 or 3, wherein at least one of the pair of jaw members is coupled to a source of electrical energy (54).

5. The surgical instrument according to claim 1, wherein:
the tensioning mechanism is configured to impart a variable force to the pair of articulating cables to constantly maintain the pair of articulating cables in tensile state, the variable force dependent upon a variable length of the pair of articulating cables.

6. The surgical instrument according to any preceding claim, wherein the tensioning mechanism includes a spring (104) operatively coupled to the pair of articulating cables to transmit a force to the pair of articulating cables, and wherein the spring is configured to change in length in response to a change in length of the pair of articulating cables.

7. The surgical instrument according to claim 6, wherein the spring comprises a compression spring coupled between a stationary base hub (102) and a movable component (106) of the articulation drive mechanism to impart a variable force to the movable component.

8. The surgical instrument according to claim 7, wherein the movable component comprises a first collar (86, 210) coupled to the pair of articulating cables, and wherein the first collar is longitudinally movable to induce the distal articulating portion of the elongated shaft to pivot.

9. The surgical instrument according to claim 8, wherein the articulation drive mechanism includes a second collar (86, 212) longitudinally movable in response to pivotal motion of the distal articulating portion of the elongated shaft.

## Patentansprüche

1. Chirurgisches Instrument (10), umfassend:
ein Gehäuse (12), das einen länglichen Schaft (18) aufweist, der sich distal davon erstreckt, wobei der längliche Schaft einen Endeffektor (16) zum Behandeln von Gewebe stützt;
mindestens ein spannbares Element (66, 68), das sich mindestens teilweise durch den länglichen Schaft erstreckt, wobei ein distales Ende des mindestens einen spannbaren Elements funktionsbereit an den Endeffektor gekoppelt ist, sodass eine Längsbewegung in dem mindestens einen spannbaren Element eine Bewegung des Endeffektors veranlasst;
einen Antriebsmechanismus (80), der funktionsbereit an ein proximales Ende des mindestens einen spannbaren Elements gekoppelt ist, wobei der Antriebsmechanismus einen Aktuator (40) einschließt, der funktionsbereit ist, um eine Längsbewegung in dem mindestens einen spannbaren Element zu veranlassen; und
einen Spannmechanismus, der konfiguriert ist, um konstant eine proximal ausgerichtete Kraft auf den Antriebsmechanismus auszuüben, sodass die proximal ausgerichtete Kraft auf das mindestens eine spannbare Element übertragen wird, **dadurch gekennzeichnet, dass**
der längliche Schaft einen proximalen Abschnitt (20), der sich distal von dem Gehäuse erstreckt, und einen distalen artikulierenden Abschnitt (22), der sich distal von dem proximalen Abschnitt erstreckt, einschließt, wobei der distale artikulierende Abschnitt mindestens ein Gelenk darin definiert, um zu erlauben, dass sich der distale artikulierende Abschnitt in Bezug auf den proximalen Abschnitt des länglichen Schafts dreht,
das mindestens eine spannbare Element ein Paar Artikulationskabel (66, 68) einschließt, das funktionsbereit an den Endeffektor gekoppelt ist, sodass eine relative Längsbewegung zwischen den Artikulationskabeln eine Artikulation des Endeffektors veranlasst,
der Endeffektor ein Paar Klemmbackenelemente (30, 32) einschließt,
das chirurgische Instrument weiter ein Antriebskabel (70) einschließt, das sich mindestens teilweise durch den verlängerten Schaft erstreckt und funktionsbereit ist, um mindestens eines des Paares Klemmbackenelemente zwischen einer offenen Position, in der das Paar Klemmbackenelemente im Wesentlichen voneinander beabstandet ist, und einer geschlossenen Position, in der das Paar Klemmbackenelemente näher beieinander liegt, zu bewegen und
die proximal ausgerichtete Kraft des Spannmechanismus auf das Paar Artikulationskabel übertragen wird, um trotz einer Kriechdehnung des Paares Artikulationskabel eine konstante Spannung auf das Paar Artikulationskabel zu halten.

2. Chirurgisches Instrument nach Anspruch 1, wobei der Spannmechanismus eine Basisnabe (102), die in einer festen Position an das Instrument gekoppelt ist, und eine Feder (104), die zwischen der Basisnabe und dem Antriebsmechanismus gekoppelt ist, einschließt, um die proximal ausgerichtete Kraft auf den Antriebsmechanismus auszuüben.

3. Chirurgisches Instrument nach Anspruch 1 oder 2, wobei der Antriebsmechanismus erste und zweite Manschetten (210, 212) einschließt, die an ein entsprechendes Artikulationskabel gekoppelt sind, und wobei die Feder die erste Manschette trägt.

4. Chirurgisches Instrument nach Anspruch 1, 2 oder 3, wobei mindestens eines des Paares Klemmbackenelemente an eine Quelle elektrischer Energie (54) gekoppelt ist.

5. Chirurgisches Instrument nach Anspruch 1, wobei:
der Spannmechanismus konfiguriert ist, um eine variable Kraft auf das Paar Artikulationskabel auszuüben, um das Paar Artikulationskabel konstant im spannbaren Zustand zu halten, wobei die variable Kraft von einer variablen Länge des Paares Artikulationskabel abhängt.

6. Chirurgisches Instrument nach einem der vorstehenden Ansprüche, wobei der Spannmechanismus eine Feder (104) einschließt, die funktionsbereit an das Paar Artikulationskabel gekoppelt ist, um eine Kraft auf das Paar Artikulationskabel zu übertragen, und wobei die Feder konfiguriert ist, um in Reaktion auf eine Veränderung der Länge des Paares Artikulationskabel seine Länge zu verändern.

7. Chirurgisches Instrument nach Anspruch 6, wobei die Feder eine Druckfeder umfasst, die zwischen einer ortfesten Basisnabe (102) und einer beweglichen Komponente (106) des Artikulationsantriebsmechanismus gekoppelt ist, um eine variable Kraft auf die bewegliche Komponente auszuüben.

8. Chirurgisches Instrument nach Anspruch 7, wobei die bewegliche Komponente eine erste Manschette (86, 210) umfasst, die an das Paar Artikulationskabel gekoppelt ist, und wobei die erste Manschette in Längsrichtung beweglich ist, um zu veranlassen, dass sich der distale artikulierende Abschnitt des länglichen Schafts dreht.

9. Chirurgisches Instrument nach Anspruch 8, wobei der Artikulationsantriebsmechanismus eine zweite Manschette (86, 212) einschließt, die in Reaktion auf die Drehbewegung des distalen artikulierenden Abschnitts des länglichen Schafts in Längsrichtung beweglich ist.

## Revendications

1. Instrument chirurgical (10), comprenant :
un boîtier (12) ayant un arbre allongé (18) s'étendant de manière distale depuis celui-ci, l'arbre allongé supportant un effecteur d'extrémité (16) pour traiter un tissu ;
au moins un élément de traction (66, 68) s'étendant au moins partiellement à travers l'arbre allongé, une extrémité distale de l'au moins un élément de traction étant fonctionnellement couplée à l'effecteur d'extrémité de telle sorte qu'un mouvement longitudinal dans l'au moins un élément de traction provoque un déplacement de l'effecteur d'extrémité ;
un mécanisme d'entraînement (80) fonctionnellement couplé à une extrémité proximale de l'au moins un élément de traction, le mécanisme d'entraînement incluant un actionneur (40) pouvant fonctionner pour provoquer un mouvement longitudinal dans l'au moins un élément de traction ; et
un mécanisme de mise en tension configuré pour conférer de manière constante une force dirigée de manière proximale sur le mécanisme d'entraînement de telle sorte que la force dirigée de manière proximale est transmise à l'au moins un élément de traction, **caractérisé en ce que**
l'arbre allongé inclut une partie proximale (20) s'étendant de manière distale depuis le boîtier et une partie d'articulation distale (22) s'étendant de manière distale depuis la partie proximale, la partie d'articulation distale définissant au moins un raccord dans celle-ci pour permettre à la partie d'articulation distale de pivoter par rapport à la partie proximale de l'arbre allongé,
l'au moins un élément de traction inclut une paire de câbles d'articulation (66, 68) fonctionnellement couplés à l'effecteur d'extrémité de telle sorte qu'un déplacement longitudinal relatif entre les câbles d'articulation provoque l'articulation de l'effecteur d'extrémité,
l'effecteur d'extrémité inclut une paire d'éléments de mors (30, 32),
l'instrument chirurgical inclut en outre un câble d'entraînement (70) s'étendant au moins partiellement à travers l'arbre allongé et pouvant fonctionner pour déplacer au moins l'un de la paire d'éléments de mors entre une position ouverte dans laquelle la paire d'éléments de mors sont sensiblement espacés l'un de l'autre et une position fermée dans laquelle la paire d'éléments de mors sont plus proches l'un de l'autre, et
la force dirigée de manière proximale du mécanisme de mise en tension est transmise à la paire de câbles d'articulation pour maintenir une tension constante sur la paire de câbles d'articulation en dépit de tout allongement par fluage de la paire de câbles d'articulation.

2. Instrument chirurgical selon la revendication 1, dans lequel le mécanisme de mise en tension inclut un moyeu de base (102) couplé à l'instrument dans une position fixe et un ressort (104) couplé entre le moyeu de base et le mécanisme d'entraînement pour conférer la force dirigée de manière proximale sur le mécanisme d'entraînement.

3. Instrument chirurgical selon la revendication 1 ou 2, dans lequel le mécanisme d'entraînement inclut des premier et second colliers (210, 212) couplés à un câble d'articulation respectif, et dans lequel le ressort repose sur le premier collier.

4. Instrument chirurgical selon la revendication 1, 2 ou 3, dans lequel au moins l'un de la paire d'éléments de mors est couplé à une source d'énergie électrique (54).

5. Instrument chirurgical selon la revendication 1, dans lequel :
le mécanisme de mise en tension est configuré pour conférer une force variable à la paire de câbles d'articulation afin de maintenir de manière constante la paire de câbles d'articulation à l'état de traction, la force variable dépendant d'une longueur variable de la paire de câbles d'articulation.

6. Instrument chirurgical selon l'une quelconque des revendications précédentes, dans lequel le mécanisme de mise en tension inclut un ressort (104) fonctionnellement couplé à la paire de câbles d'articulation pour transmettre une force à la paire de câbles d'articulation, et dans lequel le ressort est configuré pour changer en longueur en réponse à un changement de longueur de la paire de câbles d'articulation.

7. Instrument chirurgical selon la revendication 6, dans lequel le ressort comprend un ressort de compression couplé entre un moyeu de base stationnaire (102) et un composant mobile (106) du mécanisme d'entraînement d'articulation pour conférer une force variable au composant mobile.

8. Instrument chirurgical selon la revendication 7, dans lequel le composant mobile comprend un premier collier (86, 210) couplé à la paire de câbles d'articulation, et dans lequel le premier collier est longitudinalement mobile pour provoquer le pivotement de la partie d'articulation distale de l'arbre allongé.

9. Instrument chirurgical selon la revendication 8, dans lequel le mécanisme d'entraînement d'articulation inclut un second collier (86, 212) longitudinalement mobile en réponse à un mouvement de pivotement de la partie d'articulation distale de l'arbre allongé.
